# EUROPEAN PATENT APPLICATION

(11) **EP 0 616 818 A2**
(43) Date of publication of application: **28.09.1994**
(21) Application number: 94302189.9
(22) Date of filing: 28.03.1994
(51) Int. Cl.: A61N 1/30, A61N 1/32

(54) **Electrical, drug introducing apparatus**

(30) Priority: 26.03.1993 JP 68743/93
(71) Applicant: TECHNOLOGY RESEARCH ASSOCIATION OF MEDICAL AND WELFARE APPARATUS, Tokyo (JP)
(72) Inventor: Kyuichi, Yukio, c/o Unitika Ltd., Uji-shi, Kyoto (JP); Kawaide, Akihito, c/o Unitika Ltd., Uji-shi, Kyoto (JP); Yamamoto, Keiichi, c/o Unitika Ltd., Uji-shi, Kyoto (JP); Sakamoto, Izumi, c/o Unitika Ltd., Uji-shi, Kyoto (JP)
(74) Representative: Davies, Christopher Robert

(57) **Abstract**

Apparatus for introducing a drug into a patient comprises a timer switch mechanism (2) for sequentially switching an applied voltage from a DC power source 1 back and forth between two or more electrode pairs (3,4;5,6) attached to the patient's skin (9). One electrode (3,5) of each pair contains the drug, the drug passing into the patient on the application of a voltage across the electrode pair. The voltage is cut off from one pair before the establishment of a polarisation effect between the electrodes of that pair (which would normally inhibit transmission of the drug), whilst at the same time ensuring continuous drug deliver by applying the voltage to another pair.

The drug delivery rate is increased, whilst inhibiting skin damage.

## Description

The present invention relates to electrical drug introducing apparatus, and, more particularly, to electrical drug introducing apparatus able to introduce a drug through the skin highly efficiently, whilst preventing skin damage. The invention may act as a drug delivery system.

Electrical drug introducing apparatus is now attracting general attention as a non-invasive drug administration method, and various studies are being conducted into it. In this method, however, application of a DC voltage to the skin causes polarization due to the chemical constituents of the skin, and, in this state, almost no drug delivery current flows.

To solve this problem, apparatus having a depolarization function has been proposed. For example, the use of a switch for short-circuiting both the electrodes, or a mechanism for applying reverse-polarity pulses between both electrodes, during a downtime of drug administration, is discosed in Japanese Patent Provisional Publication No 60-156,475. Japanese Patent Provisional Publication No. 64-11,565 discloses a method of reducing the electrical resistance of skin by applying a voltage of 5 to 20 V for 1 to 60 seconds by means of a constant voltage device, and of then applying a voltage of 0 to 5 V, thereby increasing the drug absorption rate. Japanese Patent Provisional Publication No. 3-45,272 covers a method of suppressing polarization by applying an AC voltage of a prescribed frequency between the electrodes

In spite of the improvements described above, a problem still exists in that the drug is not introduced at all to the patient during depolarization, because depolarization is effected during stoppage of drug delivery. Therefore, the drug delivery rate is limited, and the conventional apparatus is not suitable for administration of a drug in situations where an immediate effect is required. Further, when using a high voltage, the occurrence of skin disease is dramatically increased, even with a high-frequency pulse.

The present invention aims to provide electrical drug introducing apparatus which solves the above-mentioned problems, permits an increase in the drug introduction rate and inhibits skin impairment.

With a view to solving the above-mentioned problems, the present invention provides an electrical drug introducing apparatus in which a drug is caused to penetrate into a patient's body by applying a voltage between an electrode pair, at least one electrode of which contains the drug, the apparatus being provided with a plurality of such electrode pairs. In a preferred embodiment, the apparatus is provided with-a timer switch mechanism for sequentially switching an applied voltage from one electrode pair to another.

Embodiments of the present invention will now be described, by way of example only, with reference to the accompanying drawings, in which:
Fig. 1 shows a block diagram of electrical drug introducing apparatus according to a first embodiment of the present invention;
Fig. 2 shows a perspective view of apparatus according to the first embodiment;
Fig. 3 shows schematically the apparatus attached to a human body;
Fig. 4 shows diagrams of voltage waveforms supplied to the electrode pairs by a DC power source of the first embodiment;
Fig. 5 shows a block diagram of apparatus according to a second embodiment of the present invention;
Fig. 6 shows diagrams of voltage waveforms supplied to the electrode pairs by a DC power source of the second embodiment;
Fig. 7 shows a block diagram of conventional electrical drug introducing apparatus used in a comparative example; and
Fig. 8 shows a diagram of the voltage waveform supplied to the electrode pair in the comparative example by a DC power source.

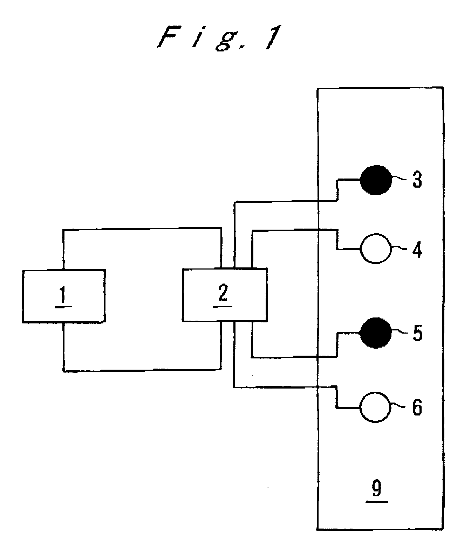

Fig. 1 is a full configurational block diagram illustrating an embodiment of the present invention provided with two pairs of electrodes.

In Fig. 1, electrodes 3 and 5 contain a drug, and counter-electrodes 4 and 6 do not. A voltage from a DC power source 1 is applied between the electrodes 3 and 4, and the drug is introduced from the electrode 3 into a patient's body 9.

In the Fig.1 embodiment, the DC voltage is switched over to be applied across electrodes 5 and 6 by means of a timer switch 2 before polarization starts between electrodes 3 and 4. Thus, introduction of the drug from electrode 3 is discontinued, whilst introduction of the drug from electrode 5 is begun. Similarly, the voltage is switched back over to be applied between electrodes 3 and 4 by means of the timer switch 2 before polarization starts between the electrodes 5 and 6, and so introduction of the drug from the electrode 5 is discontinued, whilst introduction of the drug from electrode 3 occurs again. Subsequently, the drug is introduced in succession, while avoiding polarization, by alternately switching the applied voltage between the electrodes 3-4 and 5-6 at desired switchover intervals.

When there are three or more electrode pairs, it is possible to introduce the drug sequentially from a plurality of positions by sequentially switching over voltage application from one electrode pair to another.

Of course, any electrode pairs may be employed so long as at least one electrode of each pair comprises a portion for the application of voltage and a reservoir portion capable of containing, for example, an aqueous solution of a therapeutic drug.

The applied voltage may be DC, pulsed DC or AC, but is preferably not high enough to cause skin impairment.

The frequency of the pulsed DC voltage and the AC voltage need not be particularly limited, but is preferably within a range of 1 Hz to 200 kHz.

The current between the electrodes need not be particularly limited, but is preferably within a range of 0.01 to 20 mA, or, more preferably, 0.1 to 5 mA.

The switchover interval need not be particularly limited, and may depend upon the drug and energizing conditions, but is preferably within a range of 0 to 60 minutes.

The conditions, including the kinds of voltage, frequency, current and switchover interval, as described above, may be uniform for all the electrode pairs or may be different between pairs.

Any voltage application sequence to the individual electrode pairs may be used.

When using a timer switch, any switch which has a timer giving variable switchover intervals, and permits switchover of circuits by means of this timer may be used.

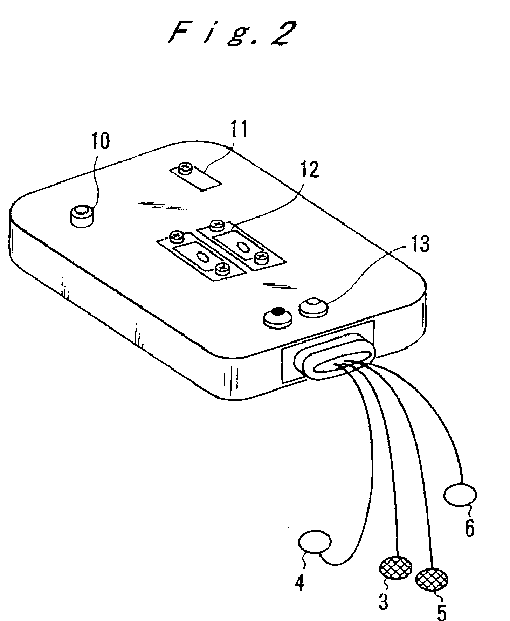

Fig. 2 is an exterior view of drug introducing apparatus provided in accordance with Fig. 1.

In Fig. 2, the electrodes 3 and 5 contain a drug and the counter-electrodes 4 and 6 do not. By previously setting a desired current value with a current setting dial 10 and turning on a power switch 11, a set voltage is applied, from a DC power source within the apparatus, between the electrodes 3 and 4, and the drug is introduced from the electrode 3 into the patient's body. If a time point at which polarization is started between the electrodes 3 and 4 is set in advance, the timer switch 2 switches over the applied voltage to be between the electrodes 5 and 6 before polarization starts between electrodes 3 and 4, and so drug introduction is discontinued from electrode 3 and begun from electrode 5. Further, before polarization starts between electrodes 5 and 6, the timer switch 2 switches the applied voltage back to be between the electrodes 3 and 4 at intervals set in the switchover timer 12, so that drug introduction is discontinued from electrode 5 and begun again from electrode 3. The drug is thus introduced in succession, while avoiding polarization, by alternately switching over voltage application between the electrode pairs 3-4 and 5-6 at desired intervals.

Power indication lamps 13 indicate the electrode pair to which the voltage is applied. If the power indication lamps 13 for the electrode pairs 3-4 and 5-6 are expressed as CH1 and CH2, respectively, CH1 lights when the voltage is applied to electrode pair 3-4, and CH2 lighted when the voltage is applied to electrode pair 5-6.

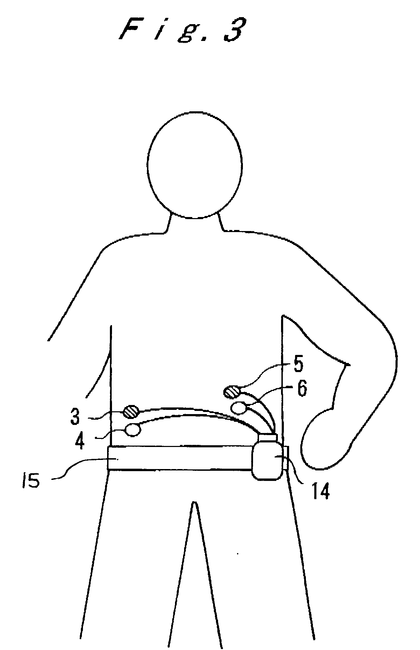

Fig. 3 illustrates an embodiment in which a drug is introduced through the hypogastrium using apparatus provided with two pairs of electrodes.

The electrical drug introducing apparatus 14 is secured to the loins by a securing belt 15 to affix electrodes 3 and 5 containing the drug, and counter-electrodes 4 and 6 not containing the drug, to the hypogastrium.

A desired current value is set with a current setting dial 10, and a voltage application switchover time for the two electrode pairs is set on the application switchover timer 12. Then, by turning on the power switch 11, voltage is applied between the electrodes 3 and 4, and the drug is introduced from electrode 3 into the hypogastrium.

After the lapse of the time set in the switchover timer 12, the applied voltage is switched over by the timer switch 2 from the electrode pair 3-4 to 5-6, and the drug is introduced from electrode 5 into the hypogastrium.

Thereafter, as the voltage is switched back and forth between the electrode pairs 3-4 and 5-6, the drug is introduced into the hypogastrium successively by one pair and then the other.

Drugs applicable in the present invention are not limited in kind, and any appropriate one may be used. For cancer treatment, for example, any of such antitumour agents as cisplatin, mitomycin C and adriamycin may favourably be used.

To adjust blood pressure, any of such antihypertensive drugs as reserpine, guanethidine sulfate, chlorothiadid and hydrochlorothiadid and such presser drugs as norepinephrine may favorably be used.

For treatment of arhythmia and heart diseases and for adjustment of heart functions and pulse wave, any of such cardiotonic drugs as digitoxin uabyne, and such arhythomia treating drugs as guinidine sulfate, procaine amid hydrochloride and propranolol may favourably be used.

To adjust body temperature, and for antipyretic and analgesic purposes, any of such antipyretic and analgesic drugs as sodium solicylate, aspirin and acetoaminophenon may favorably be used.

For adjustment of water in the body and diuretic purposes, any of such diuretic drugs as azocemide, chlocemide, acetoazolamide and soldactone may favourably be used.

To treat urinary incontinency, any drugs permitting control of contraction and relaxation of bladder muscle or urethra sphincter such as telodirine hydrochloride, oxybuthynine hydrochloride, flaboxat hydrochloride and wubrethide may favourably be used.

To treat diabetes mellitus, any of such peptide drugs as insulin may favourably be used.

After dissolving or dispersing any one or more of these drugs in water or an aqueous solution with a pH adjusted to a desired value, the drug reservoirs in the electrodes may be charged with the resultant solution. Different electrodes may be charged with different kinds of drug or with a single kind of drug.

The drug concentration may be arbitrary. For example, the solution may be used as a dispersion liquid by increasing the concentration beyond the solubility. It is desirable, however, to appropriately adjust the concentration in response to the required skin penetration rate.

The body portion to which the electrodes are affixed is not particularly limited, but preferably the electrodes are applied to the upper limb, lower extremity, abdomen or loins.

The distance between the electrode containing the drug and its counter-electrode not containing the drug is not particularly limited, but is preferably within a range of 0.5 to 10 cm.

The present invention will now be described in further detail by means of some examples.

### EXAMPLE 1

Insulin was dissolved to a concentration of 10 mg/ml in distilled water, and electrodes 3 and 5, of an electrical drug introducing apparatus provided with two pairs of electrodes as shown in Fig. 1, were charged with 1.5 ml of this solution. These electrodes, together with electrodes 4 and 6, were attached to the dorsum of a shaved hairless rat (male, 200g) having high bloodsugar. The positive pole was connected to the electrodes 3 and 5, and the negative pole to the electrodes 4 and 6. An electric current of 5 mA was fed from a DC power source 1 such as a dry battery for a total of 120 minutes, while alternately switching over between the electrode pairs 3-4 and 5-6. Switchover between the individual electrode pairs was effected with the use of a timer switch 2 at intervals of 0.05 seconds.

As a result. the bloodsugar value significantly decreased, and no noticeable skin damage was observed on the portion to which the electrodes were attached, in spite of the use of high current.

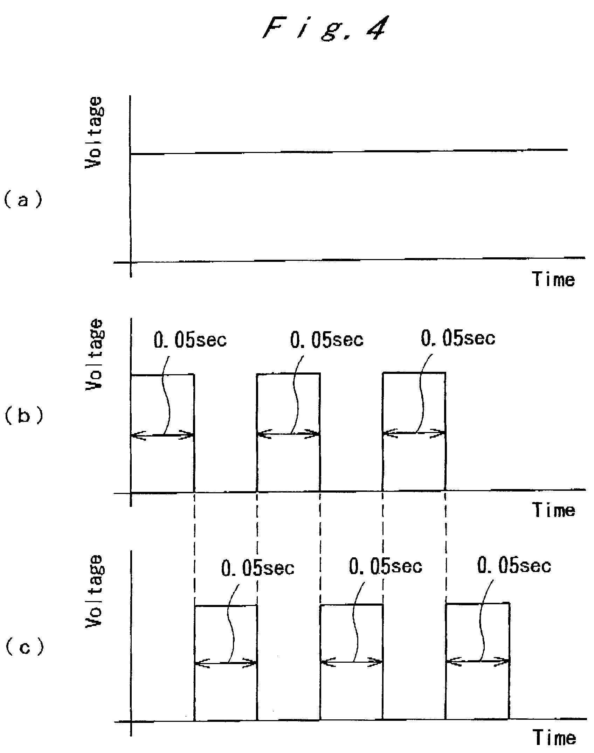

Fig 4(a) illustrated the voltage waveform supplied by the DC power source in this example, and Figs. 4(b) and 4(c) show voltage waveforms supplied to the electrode pairs 3-4 and 5-6.

### EXAMPLE 2

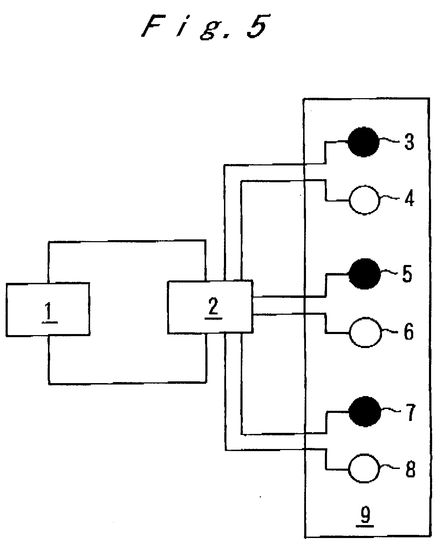

Another pair of electrodes was added to the apparatus of Example 1, as shown in Fig. 5, and a drug was introduced under the same conditions as in Example 1, except that the current was supplied for 90 minutes.

Despite the short period of supply of electric current, the bloodsugar value of the high-bloodsugar hairless rat decreased significantly. No skin damage was observed on the portion to which the electrodes were attached.

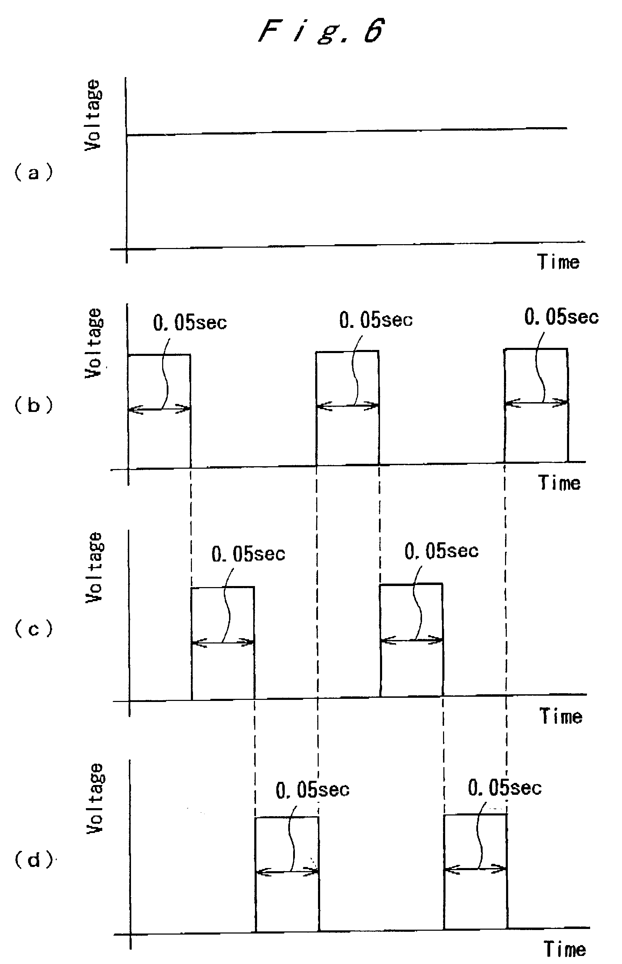

Fig 6(a) illustrates the voltage waveform supplied by the DC power source in this example, and Figs. 6(b), 6(c) and 6(d) show voltage waveforms supplied to the electrode pairs 3-4, 5-6 and 7-8.

### EXAMPLE 3, COMPARATIVE EXAMPLE 1

Using the appratus of Example 2, the electrodes 3, 5 and 7 were charged with oxybuthynine hydrochloride and were attached, together with the electrodes 4, 6 and 8, to the dorsum of a shaved hairless rat (male, 225g). The positive pole was connected to the electrodes 3, 5 and 7, and the negative pole to the electrodes 4, 6 and 8. Electric current of 2 mA was fed for 60 minutes in total, while switching between the electrode pairs 3-4, 5-6 and 7-8. Voltage switchover to the individual electrode pairs was effected at intervals of 10 minutes.

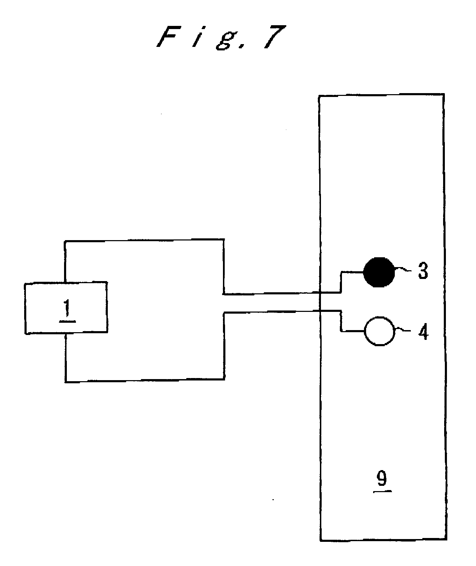

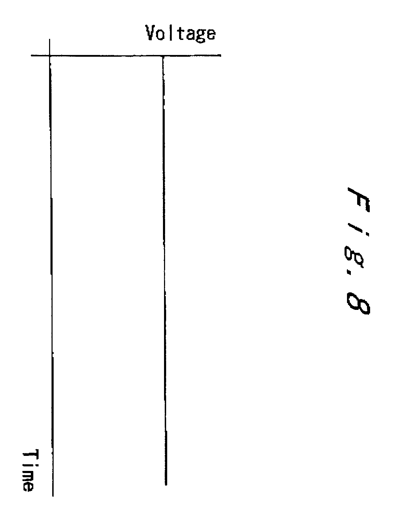

For comparison purposes, a conventional electrical drug introducing apparatus shown in Fig. 7, and provided with only a single pair of electrodes, was also used. An electrode 3 was charged with oxybuthynine hydrochloride, and was attached, together with an electrode (4), to the dorsum of a shaved hairless rat (male, 225g). The positive pole was connected to the electrode (3), and the negative pole to the electrode 4. An electric current of 2 mA was fed for 60 minutes. Fig. 8 illustrates the voltage waveform supplied by the DC power source to the electrical drug introducing apparatus.

In all cases, blood was sampled at prescribed intervals of time after administration. After obtaining serum through centrifugal separation (2,000 rpm for 10 minutes), the drug concentration in serum was measured by gas chromatography. The results are shown in Table 1.

As is clear from Table 1, a significant increase in the concentration in blood was observed when using apparatus according to the present invention.

**Table 1**

| Time of supply of current (minutes) | Concentration in plasma (ng/ml) | |
|---|---|---|
| | Example 3 | Comp. Example 1 |
| 10 | 345 | 133 |
| 30 | 775 | 250 |
| 60 | 1,286 | 401 |

By using apparatus according to the present invention, it is possible to introduce a drug, while avoiding polarization, by applying a voltage to sequentially switched over electrode pairs, with a remarkably increased amount of introduced drug per unit time.

Since the drug is introduced from a plurality of points, it is possible to introduce the drug in a required amount even with low current, or when applying high current, in a short period of time, with minimized skin damage.

It is furthermore possible to use this apparatus to administer a drug in situations in which an immediate effect is required (which has previously been considered difficult), and to eliminate discomfort caused by injection. It is effective as a method of administering a drug to a patient to whom oral administration is not applicable.

## Claims

1. Electrical drug introducing apparatus comprising a plurality of electrode pairs, each pair including at least one electrode containing a drug which is caused to penetrate into the body of a patient by applying a voltage across that electrode pair.

2. Electrical drug introducing apparatus as claimed in claim 1, wherein the apparatus is provided with a timer switch mechanism for applying a voltage across each of said plurality of electrode pairs by sequentially switching over.

3. Apparatus for introducing a drug into a patient by applying a voltage across a pair of electrodes, with at least one of the electrodes in the pair being adapted to carry the drug, wherein the apparatus is provided with a plurality of such electrode pairs, and comprises means for applying a voltage to at least one of the electrode pairs and for subsequently cutting off the voltage to that pair whilst continuing the administration of the drug through the use of another electrode pair.

4. Apparatus according to Claim 3, wherein the applied voltage is cut off from an electrode pair before the establishment to any substantial degree of a polarisation state between the electrodes of that pair.

5. Apparatus according to Claim 3 or 4, wherein the voltage is cut off after a predetermined time period.

6. Apparatus according to Claim 3,4 or 5, wherein the apparatus includes switching means for switching an applied voltage from one electrode pair to another.

7. Apparatus according to Claim 6, wherein switching is carried out sequentially between each electrode pair.

8. Apparatus according to any preceding claim, wherein a voltage may be applied between a first drug-containing electrode and a second electrode at one stage of the drug administration, and between the same drug-containing electrode and a different second electrode at another stage.

9. A method of controlling apparatus for administering a drug to a patient by the application of a voltage across two or more electrode pairs, at least one of the electrodes in each pair containing the drug, wherein not all of the pairs receive a voltage at any one time, and wherein the pair or pairs to which a voltage is applied is or are selected to ensure the continuous supply of the drug to the patient and to facilitate drug administration.

10. A method according to Claim 9, wherein the voltage applied to a pair is removed before the establishment of a polarisation state between the electrode pair to any significant degree.

11. A method according to Claim 9 or 10, wherein the voltage applied to a pair is removed after a predetermined time period.
